(19) Europäisches Patentamt
European Patent Office
Office européen des brevets

(11) Publication number: 0 194 831 A1

# (12) EUROPEAN PATENT APPLICATION

(21) Application number: 86301679.6

(22) Date of filing: 10.03.86

(51) Int. Cl.[4]: C 07 C 141/10
C 07 C 139/00

(30) Priority: 11.03.85 US 710748

(43) Date of publication of application: 17.09.86 Bulletin 86/38

(84) Designated Contracting States: BE DE FR GB

(71) Applicant: THE PROCTER & GAMBLE COMPANY
One Procter & Gamble Plaza
Cincinnati Ohio 45202(US)

(72) Inventor: Corrigan, Patrick Joseph
145 Twin Lakes Drive
Fairfield Ohio 45014(US)

(72) Inventor: Hawkins, Larry Nelson
4045 Glen Este-Withamsville Road
Cincinnati Ohio 45245(US)

(74) Representative: Gibson, Tony Nicholas et al,
Procter & Gamble (NTC) Limited Whitley Road
Longbenton Newcastle upon Tyne NE12 9TS(GB)

**(54) Improved process for preparation of sulfuric esters of higher alcohols.**

(57) Monounsaturated fatty alcohols are sulfated with minimal reaction at the double bond by using a multistage sulfation process in which the sulfating agent is chlorosulfonic acid, or a dioxane complex of chlorosulfonic acid or sulfur trioxide. The sulfation is carried out in a refluxing solvent medium, followed by ammonia or amine neutralization at each stage.

EP 0 194 831 A1

Croydon Printing Company Ltd.

# IMPROVED PROCESS FOR PREPARATION OF SULFURIC ESTERS OF HIGHER ALCOHOLS

Patrick J. Corrigan
Larry N. Hawkins

## FIELD OF THE INVENTION

This invention pertains to the sulfation of higher monounsaturated fatty alcohols, e.g., oleyl alcohol, to produce the sulfuric esters of said alcohols. The sulfuric esters, when neutralized with a base such as sodium hydroxide are useful as synthetic detergents.

## BACKGROUND OF THE INVENTION

The salts of the sulfuric esters of higher monounsaturated fatty alcohols, e.g., sodium oleyl sulfate, are highly desirable as synthetic detergents. Because of their long hydrocarbon chain, they have better detergency properties than the corresponding shorter chain saturated alcohol sulfates (e.g., sodium lauryl sulfate); and because of their unsaturation, they are more watersoluble than the corresponding long chain saturated alcohol sulfates (e.g., sodium stearyl sulfate). Thus, the unsaturated long chain alcohol sulfates combine the desirable solubility properties of the short chain saturated alcohol sulfates and the desirable detergency properties of the long chain saturated alcohol sulfates. This combination of properties makes the long chain monounsaturated fatty alcohol sulfates particularly desirable for use in formulating concentrated liquid detergents and in the formulation of detergents which are to be used for laundering or other detergency operations in cool water.

Because of the inherent reactivity of double bonds, sulfation of unsaturated alcohols by conventional methods, using common sulfating agents such as sulfur trioxide or chlorosulfonic acid, results in a substantial amount of reaction at the double bond site. This results in the product of the sulfation reaction having poorer detergency properties and dark color.

The prior art discloses attempts to deal with this problem. U.S. Pat. No. 2,099,214, McAllister, issued Nov. 6, 1967, discloses a process for sulfating unsaturated alcohols with a sulfating reagent which is an addition product of dioxane and either sulfur trioxide or chlorosulfonic acid. U.S. Pat. No. 3,763,208, Sowerby, issued Oct. 2, 1973, discloses a stepwise process for sulfating unsaturated alcohols wherein the alcohol is reacted with substoichiometric amount of sulfur trioxide, followed by neutralization of the sulfated material with an amine (or ammonia), and then followed by addition of more sulfur trioxide, and neutralization with amine. The fully sulfated, amine-neutralized reaction product can then be converted to the sodium salt by reaction with sodium hydroxide.

It is an objective of the present invention to provide a further improved process for sulfation of monounsaturated fatty alcohols to produce detergent grade monounsaturated fatty alcohol sulfate salts.

## SUMMARY OF THE INVENTION

According to the present invention there is provided a multistage process for sulfating a fatty alcohol feedstock which comprises at least 40% of one or more $C_{18}$-$C_{22}$ monounsaturated fatty alcohols, said process comprising:

A. a first stage comprising the steps of:

(i) reacting the alcohol feedstock with a sulfating agent selected from chlorosulfonic acid, 1,4-dioxane-complexed chlorosulfonic acid and 1,4-dioxane-complexed sulfur trioxide, the amount of said sulfating agent being from 10% to 40% of that which is stoichiometrically equivalent to the total amount of alcohol present in the feedstock, said reaction being conducted under substantially anhydrous conditions in a closed vessel equipped with refluxing means, said reaction being conducted at a temperature of from 20°C to 40°C in a solvent which is inert to the components of the reaction mix and which has a boiling point of from 20°C to 90°C at 760 mm Hg, said reaction being conducted at a subatmospheric pressure selected such that the solvent refluxes at the reaction temperature; and

(ii) neutralizing the sulfated alcohol from Step (i) with a base selected from ammonia and primary, secondary and tertiary amines;

B. from zero to four intermediate stages wherein in each of said intermediate stages the steps of Stage A are repeated, except that in each of said intermediate stages the amount of sulfating agent employed is from 10% to 50% of that which is stoichiometrically equivalent to the total amount of unreacted alcohol remaining in the reaction product after completion of the immediately preceding stage; and

C. a final stage in which the steps of Stage A are repeated, except that in said final stage the amount of sulfating agent employed is from 100% to 120% of that which is stoichiometrically equivalent to the total amount of alcohol remaining in the reaction product after completion of the immediately preceding stage.

## DESCRIPTION OF THE PREFERRED EMBODIMENTS

According to the present invention it has been found that by carrying out the sulfation of long chain unsaturated fatty alcohols in a multistage process employing specific sulfating reagents and solvents under reflux conditions, and amine-neutralization of the sulfated alcohols at each stage, reaction at the double bond site of the alcohols and discoloration of the reaction product can be minimized.

## The Alcohols

The alcohols which it is desired to sulfate according to the present process are the monounsaturated fatty alcohols containing from 18 to 22 carbon atoms. Examples of such alcohols are 9-octadecen-1-ol (oleyl alcohol), 11-eicosen-1-ol and 13-docosen-1-ol (erucyl alcohol). The most important of these, from the standpoint of preparing a highly desirable alkyl sulfate surfactant product is oleyl alcohol and the present invention will be described primarily with reference to sulfation of oleyl alcohol. However, it will be understood that the invention is applicable to the entire range of $C_{18}$-$C_{22}$ monounsaturated alcohols.

These alcohols are commercially produced by conversion of the natural fats and oils in which they occur. For example, tallow is converted by methanolysis to the methyl esters of tallow fatty acids and these methyl esters are converted to the corresponding alcohols by hydrogenation. The resulting product, depending on the source of the tallow, will contain from 30% to 50% oleyl alcohol, the remaining alcohols being primarily saturated alcohols. This product can then be distilled to produce a higher purity oleyl alcohol. Typically, commercial grade "oleyl alcohol" contains at least . 40%, and typically from 45% to 85%, actual oleyl alcohol. The other materials in the commercial product are primarily saturated fatty alcohols and $C_{14}$-$C_{17}$ unsaturated alcohols. Of course, by repeated distillation, an essentially pure 100% oleyl alcohol can be produced, but this is not practical or necessary for most commercial uses. For purposes of the present invention the feedstock should contain at least 40%, preferably at least 70%, and most preferably at least 80% monounsaturated $C_{18}$-$C_{22}$ fatty alcohol.

## The Sulfating Agents

The sulfating agent of the present invention can be selected from the group consisting of chlorosulfonic acid or the complexes of chlorosulfonic acid or sulfur trioxide with 1,4-dioxane. These sulfating agents are well known in the art. See, for example, U.S. Pat. No. 2,098,114, Suter, issued Nov. 2, 1937.

In the dioxane complexes, one mole of

1,4-dioxane is capable of complexing either one or two moles of chlorosulfonic acid or sulfur trioxide. For purposes of the present invention either the mono- or di- complex can be used. Mixtures of the sulfating agents of the invention can also be used. The preferred sulfating agent herein is chlorosulfonic acid.

The Solvents

The solvents suitable for use as the reaction medium in the present invention are selected from the group consisting of organic solvents which are inert to the reactants and products of the process of the invention and which have a boiling point at 760 mm Hg of from 20°C to 90°C. Examples of such solvents are chloroform, carbon tetrachloride, 1,2-dichloroethylene, methyl chloroform and methylene chloride. The preferred solvent is methylene chloride. The amount of solvent used should be from 2 to 10 times, by weight, of the total amount of alcohol to be sulfated.

The Neutralizing Base

The nitrogenous neutralizing bases used in the process herein are selected from ammonia and primary, secondary and tertiary amines. Examples of the amines are mono-, di- and trimethyl, ethyl and propyl amines and mono-, di- and tri- ethanol and propanol amines. Ammonia is the preferred neutralizing agent.

Conduct of the Reaction

The reaction of the present invention is carried out in at least two stages, each stage comprising a sulfating step and a neutralizing step. In the first stage, the amount of sulfating agent is from 10% to 40% of the amount which is stoichiometrically equivalent to the total amount of fatty alcohol in the feed. In each succeeding stage, except the last stage, the amount of sulfating agent used is 50% or less than (preferably 10% to 50% of) the amount which is stoichiometrically equivalent to the amount of fatty alcohol which is fed to that stage. In the final stage, in order to complete the sulfation of the alcohol, the amount of sulfating agent used is from 100% to 120% of the amount which is stoichiometrically equivalent to the amount of fatty alcohol in the neutralized reaction product which is fed to that stage. Chlorosulfonic acid and sulfur trioxide (whether or not complexed with dioxane) each reacts with the alcohols on a 1:1 molar basis.

In each stage of the process there are two steps, a sulfation step and a neutralization step.

The sulfation step is carried out in a closed vessel (capable of operating at subatmospheric pressure), equipped with a reflux means (e.g., a reflux condenser). The sulfation reaction is carried out at a temperature of from 20°C to 40°C at a subatmospheric pressure selected such that the solvent which is used as a reaction medium refluxes at the reaction temperature. For example, when methylene chloride is used as the solvent, the pressure will be about 500 mm at 25°C. Refluxing prevents spot overheating, which can cause discoloration of the reaction product. The sulfation should be carried out under substantially anhydrous conditions, i.e., no more than 0.01% water in the reaction mix.

In the neutralization step, ammonia or an amine is added to the sulfated reaction product of the sulfation step to neutralize the sulfated alcohol and any excess sulfating agent which may be present. Neutralization is on a 1:1 molar basis, i.e., one mole of ammonia or monoamine (i.e., amine having one nitrogen) neutralizes one mole of sulfated alcohol or sulfating agent. Neutralization of the sulfated alcohol with ammonia or amine is accompanied by a dramatic color change from purple to yellow at the end point. Pressure conditions for the neutralization step are not critical. The temperature, however, should be kept at less than 60°C to prevent discoloration of the product. Since the neutralization reaction is exothermic, use of a cooling means (e.g., a cooling bath) is usually required. Neutralization can be done under the same temperature and pressure conditions as used in the sulfation step, if desired, but this is not necessary. Atmospheric pressure and ambient temperatures can conveniently be used.

The overall process is carried out in at least two stages, each stage comprising a sulfation step and an ammonia or amine neutralization step. In the first stage from 10% to 40% (preferably from 20% to 30%) of the amount of sulfating agent which is stoichiometrically equivalent to the amount of alcohol in the alcohol feed is used. After neutralization, the first stage is followed by from zero to four intermediate stages in which the feed is the neutralized reaction product from the immediately preceding stage. The steps performed in each intermediate stage are the same as in the first stage. Preferably, there is one intermediate stage. More than four can be used, but this is generally not desirable for economic reasons. The intermediate stage(s) is followed by a final stage in which the steps are the same as in the first stage, except that the amount of sulfating agent used in the sulfating step of the final stage is an amount which is sufficient to neutralize all of the alcohol in the feed. This will be from 100% to 120% of the amount of sulfating agent which is stoichiometrically equivalent to the amount of alcohol in the feed for the final stage. Some excess over the 100% stoichiometric amount is desirable to insure completeness of reaction. The feed for the final stage, of course, is the neutralized reaction product from the immediately preceding stage.

If it is desired to use the sulfated alcohol in the form of its alkali metal salt, the amine or ammonia-neutralized product from the final stage can be reacted with an alkali metal base (e.g., NaOH) to convert it to the alkali metal salt. The solvent should be stripped from the sulfated alcohol product before use in formulation of detergents. Stripping should be done at a temperature below 60°C (using subatmospheric pressure if necessary) so as not to degrade the sulfated alcohol product.

Preferably, the process of the present invention is carried out in three stages.

A. a first stage comprising the steps of:

(i) reacting the alcohol feedstock with a sulfating agent selected from chlorosulfonic acid, 1,4-dioxane-complexed chlorosulfonic acid and 1,4-dioxane-complexed sulfur trioxide, the amount of said sulfating agent being from 10% to 40% of that which is stoichiometrically equivalent to the total amount of alcohol present in the feedstock, said reaction being conducted under substantially anhydrous conditions in a closed vessel equipped with refluxing means, said reaction being conducted at a temperature of from 20°C to 40°C in a solvent which is inert to the components of the reaction mix and which has a boiling point of from 20°C to 90°C at 760 mm Hg, said reaction being conducted at a subatmospheric pressure selected such that the solvent refluxes at the reaction temperature; and

(ii) neutralizing the sulfated alcohol from Step (i) with a base selected from ammonia and primary, secondary and tertiary amines;

B. a second stage comprising the steps of:

(i) sulfating the neutralized reaction product from Step (ii) of Stage A with a sulfating agent selected from chlorosulfonic acid, 1,4-dioxane-complexed chlorosulfonic acid and 1,4-dioxane-complexed sulfur trioxide, the amount of said sulfating agent being from 10% to 50% of that which is stoichiometrically equivalent to the total amount of alcohol present in the neutralized reaction product of Step (ii) of Stage A, said reaction being conducted in a closed vessel equipped with refluxing means, said reaction being conducted at a temperature of from 20°C to 40°C in a solvent which is inert to the components of the reaction mix and which has a boiling point of from 20°C to 90°C at 760 mm Hg, said reaction being conducted at a subatmospheric pressure selected such that the solvent refluxes at the reaction temperature; and

(ii) neutralizing the sulfated alcohol from Step (i) of Stage B with a base selected from ammonia and primary, secondary and tertiary amines; and

C. a final stage comprising the steps of:

(i) sulfating the neutralized reaction product of Step (ii) of Stage B with a sulfating agent selected from the group consisting of chlorosulfonic acid, 1,4-dioxane-complexed chlorosulfonic acid and 1,4-dioxane-complexed sulfur trioxide, the amount of said sulfating agent being from 100% to 120% of that which is stoichiometrically equivalent to the total amount of alcohol present in the neutralized reaction product of Step (ii) of Stage B, said reaction being conducted in a closed vessel equipped with refluxing means, said reaction being conducted at a temperature of from 20°C to 40°C in a solvent which is inert to the components of the reaction mix and which has a boiling point of from 20°C to 90°C at 760 mm Hg, said reaction being conducted at a subatmospheric pressure selected such that the solvent refluxes at the reaction temperature; and

(ii) neutralizing the sulfated alcohol from Step (i) of Stage C with a base selected from ammonia and primary, secondary and tertiary amines.

The invention will be illustrated by the following example.

Apparatus

A 4-port, 12 liter pyrex flask is used as the reactor. The center port is equipped with a mechanical stirrer. One side port is connected to a 500 ml acid addition flask for adding the sulfating agent. This acid addition flask can be interchanged with an ammonia sparger for neutralization of the sulfated alcohol with ammonia gas. A second side port is connected to a 750 mm long reflux condenser having approximately 500 sq. in. (3270 sq. cm) of heat transfer area. The condenser holds 3 gallons (11.4 liters) of cooling water and is operated at a flow rate of one gallon (3.785 liters) per minute using 20-25°C water. The top of the condenser is attached to a vacuum source. Between the vacuum source and the condenser are 2 cold traps and two water traps to collect any noncondensables. The third side port is equipped with a thermometer for monitoring sulfation and neutralization temperatures.

The reactor is surrounded by a warm water bath on jacks. The bath is maintained at a temperature of 40-60°C and can be raised or lowered in order to maintain the reactor at a constant temperature of 25-30°C throughout the sulfation and neutralization steps.

Reaction

The reactor is charged with 1000g (3.7 moles) of "oleyl alcohol" (Iodine Value 89.5) dissolved in 4 liters of methylene chloride. The "oleyl alcohol" is Sherex Adol 85[R], which has been further distilled to increase its actual oleyl alcohol content to 81% from the "as received" level of 78%. The temperature of this solution is brought to 25-30°C with the water bath. A vacuum of 500-600 mm Hg (absolute) is then drawn on the reactor, thereby causing the methylene chloride to boil. The methylene chloride vapors condense in the condenser and return to the reaction. The stirrer is operated at a speed which keeps the solution well mixed without creating a vortex.

3.8 moles of chlorosulfonic acid (free of $H_2O$ and organic contaminants) are dissolved in sufficient methylene chloride to form a 37% solution of chlorosulfonic acid. This solution is placed in the acid addition flask. One third (1.27 moles) is added to the reactor, dropwise, over a period of 20 minutes. As the acid is added, the color of oleyl alcohol solution changes from clear to dark purple. After completion of the addition of this first third of the acid, the contents of the reactor are allowed to mix for 2-3 minutes. The vacuum on the reactor is then broken and the contents are allowed to reach atmospheric pressure. The flask is then sparged with nitrogen to remove the last traces of HCl, so as to minimize presence of chloride salts in the finished product.

The acid addition flask is then removed and replaced with an ammonia sparger. The reaction mix is sparged with ammonia until it is completely neutralized. The neutral point can be easily determined by monitoring the color of the reaction mix. A change from purple to light yellow occurs at the neutral point.

Vacuum is then reapplied to the reactor and the sulfation/neutralization procedure described above is repeated two more times. Ammonium alkyl sulfate in a yield of 97% is obtained. The resulting product is pale yellow and has an Iodine Value of 86.3, compared to 89.5 for the original oleyl alcohol. Methylene chloride is removed by distillation, leaving a pale yellow waxy product. The amount of ammonium oleyl sulfate in the resulting ammonium alkyl sulfate product is substantially proportional to the amount of actual oleyl alcohol in the original alcohol feed, which in this case is 81%.

CLAIMS

1. A multistage process for sulfating a fatty alcohol feedstock which comprises at least 40% of one or more $C_{18}$-$C_{22}$ monounsaturated fatty alcohols, said process comprising:

A. a first stage comprising the steps of:

(i) reacting the alcohol feedstock with a sulfating agent selected from chlorosulfonic acid, 1,4-dioxane-complexed chlorosulfonic acid and 1,4-dioxane-complexed sulfur trioxide, the amount of said sulfating agent being from 10% to 40% of that which is stoichiometrically equivalent to the total amount of alcohol present in the feedstock, said reaction being conducted under substantially anhydrous conditions in a closed vessel equipped with refluxing means, said reaction being conducted at a temperature of from 20°C to 40°C in a solvent which is inert to the components of the reaction mix and which has a boiling point of from 20°C to 90°C at 760 mm Hg, said reaction being conducted at a subatmospheric pressure selected such that the solvent refluxes at the reaction temperature; and

(ii) neutralizing the sulfated alcohol from Step (i) with a base selected from ammonia and primary, secondary and tertiary amines;

B. from zero to four intermediate stages wherein in each of said intermediate stages the steps of Stage A are repeated, except that in each of said intermediate stages the amount of sulfating agent employed is from 10% to 50% of that which is stoichiometrically equivalent to the total amount of unreacted alcohol remaining in the reaction product after completion of the immediately preceding stage; and

C. a final stage in which the steps of Stage A are repeated, except that in said final stage the amount of sulfating agent employed is from 100% to 120% of that which is stoichiometrically equivalent to the total amount of alcohol remaining in the reaction product after completion of the immediately preceding stage.

2. A process according to claim 1 wherein the monounsaturated fatty alcohol is oleyl alcohol.

3. A process according to either one of claims 1 and 2 wherein the solvent is methylene chloride.

4. A process according to any one of claims 1-3 in which the number of stages in B is one.

5. A process according to any one of claims 1-4 wherein the sulfating agent is chlorosulfonic acid.

European Patent Office

# EUROPEAN SEARCH REPORT

0194831
Application number

EP 86301679.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,A | US - A - 3 763 208 (AUSTEN EDGAR SOWERBY)<br>* Claims 1,6; examples 3,4 *<br>-- | 1,2 | C 07 C 141/10<br>C 07 C 139/00 |
| D,A | US - A - 2 099 ?14 (WALTER H. MC ALLISTER)<br>* Claims *<br>-- | 1,2,5 | |
| D,A | US - A - 2 098 114 (CHESTER MERLE SUTER)<br>* Claims 1-4 *<br>-- | 1,2 | |
| A | DD - A - 135 381 (DÖRFEL, KLAUS et al.)<br>* Claims 1,2,4 *<br>-- | 1 | TECHNICAL FIELDS SEARCHED (Int. Cl 4) |
| A | DE - A - 1 493 311 (UNILEVER N.V.)<br>* Claim 1 *<br>-- | 1 | C 07 C 139/00<br>C 07 C 141/00 |
| A | DE - B - 1 274 118 (VEB DEUTSCHES HYDRIERWERK RODLEBEN)<br>* Claim *<br>---- | 1 | |

The present search report has been drawn up for all claims

| Place of search | Date of completion of the search | Examiner |
|---|---|---|
| VIENNA | 15-05-1986 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82